# EUROPEAN PATENT SPECIFICATION

(11) **EP 3 852 609 B1**
(45) Date of publication and mention of the grant of the patent: **10.04.2024**
(21) Application number: 19769800.4
(22) Date of filing: 18.09.2019
(51) Int. Cl.: A61B 5/00, A61B 5/259

(54) **ELECTRODE PATCH WITH MULTIPLE MEASUREMENT POINTS**
ELEKTRODENFLÄCHE MIT MEHREREN MESSPUNKTEN
PATCH D'ÉLECTRODE À PLUSIEURS POINTS DE MESURE

(30) Priority: 21.09.2018 EP 18196123
(43) Date of publication of application: 28.07.2021
(73) Proprietor: SmartMedics Sp. z o.o., 02-566 Warsaw (PL)
(72) Inventor: WRÓBLEWSKI, Grzegorz, 02-793 Warsaw (PL); MACIEJEWSKI, Adrian, 00-384 Warsaw (PL); KOLTOWSKI, Lukasz, 03-704 Warsaw (PL)
(74) Representative: Fuchs Patentanwälte Partnerschaft mbB
(86) International application number: PCT/EP2019/074963
(87) International publication number: WO 2020/058314

(56) References cited:
- US-A- 5 868 671
- US-A1- 2002 133 069
- US-A1- 2013 281 814
- US-A1- 2016 166 170
- US-A1- 2018 020 936

## Description

### FIELD OF THE INVENTION

The present invention relates to an electrode patch for obtaining biometric parameters of a subject, in particular for performing electrocardiography (ECG), electroencephalography (EEG), electromyography (EMG), electrooculography (EOG), microdefibrillation and/or defibrillation on a subject. Further, the present invention relates to a corresponding system including such an electrode patch, a method of manufacturing an electrode patch and a method of obtaining biometric parameters from a subject by use of an electrode patch. The electrode patch can be used for medical and non-medical applications.

### BACKGROUND OF THE INVENTION

In both the medical and non-medical field, a wide range of medical electrodes are employed to monitor electrical activities of the body of a subject, such as a patient, and/or to deliver electrical impulses to the body of a subject. For instance, in the field of cardiology, electrocardiograph monitors are commonly used to obtain information relating to the electrical activity of the heart over a period of time using electrodes placed on the skin of a patient. The electrodes detect tiny electrical potential changes on the skin that arise from the heart muscle's electrophysiological pattern of depolarization during each heartbeat.

In order to allow for interpretation of specific areas of the heart, such as inferior, lateral and anterior, multiple-lead ECG may be performed. For example, for 12-lead ECG, electrodes are applied to the skin of a subject in various positions according to medical protocols - below the breast in females and overlying the breast in males - however the relative positions are kept principally intact. In a standard 12-lead ECG configuration ten electrodes are placed on the subject's skin in predetermined positions, wherein six chest electrodes (V1-V6) are located near the heart and four electrodes representing the subject's limbs (left and right arm electrodes LA and RA, and left and right leg electrodes LL and RL). Using these electrodes, three standard limb leads and three augmented limb leads (vectors) are provided, respectively, in addition to an individual lead from each of the six chest electrodes. More precisely, the leads are provided by the voltage between or from particular electrodes as follows:
LA and RA electrodes: lead I,
LL and RA electrodes: lead II,
LL and LA electrodes: lead III,
LA and a combination of RA and LL: augmented lead vector left (aVL),
RA and a combination of LA and LL: augmented lead vector right (aVR),
LL and a combination of RA and LA: augmented lead vector foot (aVF), and
leads V1 to V6;
wherein the chest electrodes can be positioned as follows:
   V1: Fourth intercostal space to the right of the sternum,
   V2: Fourth intercostal space to the left of the sternum,
   V3: Directly between electrodes V2 and V4,
   V4: Fifth intercostal space at the left midclavicular line,
   V5: Level with lead V4 at left anterior axillary line,
   V6: Level with lead V5 at left midaxillary line;
   thus resulting in twelve measurements comprising leads I, II, III, aVR, aVL, aVF, V1, V2, V3, V4, V5, V6, with RL typically being the ground electrode.

Further, in an exemplary 18-lead ECG, six additional electrodes are placed on the subject's right side and back:
Right side electrodes:
   V3R: Directly between V1 and V4R,
   V4R: Fifth intercostal space at the right midclavicular line, and
   V5R: Level with lead V4R at right anterior axillary line;
Posterior electrodes:
   V7: Left posterior axillary line, in the same horizontal plane as V6,
   V8: Tip of the left scapula, in the same horizontal plane as V6, and
   V9: Left paraspinal region, in the same horizontal plane as V6.

These six electrodes in addition to the standard 12-lead ECG electrodes, constitute an 18-lead ECG.

To obtain a correct measurement and transmission of electrical signals, the electrodes need to be positioned properly. Furthermore, the electrodes need to be connected to an ECG monitoring system so as to enable analysis of the measurements by a user, for example by medical personnel. For this purpose, the electrodes are conventionally connected with the monitoring system via cables that realize signal transmission, wherein the electrodes are attached to the corresponding cables via a connector clip. The cables in turn can be plugged into a respective diagnostic and/or therapeutic device either individually or via a joint outlet of bundled cables.

This conventional multiple-lead configuration has a number of drawbacks. The combined weight and bulk of the electrodes, the connector clips and cables running from each of the attached electrodes is disturbing to the user or patient. Thus, such configurations often limit the user's freedom of movement, tug on the user's skin, potentially get caught in clothing and the electrodes require sufficiently strong adhesives to stay at the user's skin. Further, the weight of the cables can pull the electrodes off. Oftentimes, particularly in long-term use, electrodes may detach and thus negatively affect the measurement results. Moreover, cables are conventionally snapped onto electrodes and theoretically any cable can be snapped onto each electrode. Therefore another limitation of known electrode configurations is misplacement of cables and electrodes, which leads to inaccurate measurement results. Furthermore, the (strong) adhesives and/or the occlusive effect of the electrodes at the area of skin-contact often lead to skin irritations, rashes and/or allergies.

A further drawback is that performing an ECG may be required in emergency situations like a suspected cardiac arrest or arrhythmia. Thus, the electrode positioning and connecting needs to be done as precise and time-efficiently as possible, which is particularly challenging in emergency situations and requires well-trained medical staff. Studies show that there is a wide inter-individual and inter-group variations in the positioning of electrodes.

In corresponding guidelines, performing an 18-lead ECG is warranted during a suspected acute coronary syndrome. However, to obtain an 18-lead ECG, electrodes are currently first placed on the precordial leads on the left side and a trace is recorded. Afterwards the electrodes are transferred over to the right side and another transpositioning of the posterior leads and another trace is recorded. This approach is time consuming. Moreover, in current practice 18-lead ECG is seldom performed as the posterior electrodes are difficult to attach to the patient. This is mainly because conventional electrodes are often suction cup electrodes and when a patient is lying down the suction cup electrodes detach. This lends to underdiagnosis of ST segment elevation myocardial infarction in up to 30% of cases.

In order to save time during electrode positioning and connecting, electrode patches have been developed in which the electrodes and their respective cables or conductive connections are integrated in a patch so as to allow for a quicker and/or more precise positioning of the electrodes on a patient.

Document US 8,868,152 B2 discloses an electrode sensor array apparatus for monitoring medical signals. The apparatus includes a flexible substrate adapted for positioning relative to the torso of a patient. The flexible substrate includes a central segment defining a central axis that generally conforms to an area extending along the sternum of a patient. The flexible substrate further includes an upper segment extending traversal across the central segment and adapted to generally conform to the chest area of a patient and a lower segment extending traversal across the central segment and adapted to generally conform to the abdominal area of the patient. The apparatus further includes a medical electrode disposed on at least one of the segments and a connector in electrical communication with the medical electrode and adapted to connect to an electronic monitoring system.

Document US 7,286,865 B2 discloses a precordial pad for positioning ECG electrodes on a patient. The pad includes a sizing aid and a positioning device to support correct positioning of the pad to a patient. Obtained data is transmitted from the ECG pad by wire or wirelessly.

Further, document US 6,847,836 B1 discloses an ECG electrode chest pad particularly adapted for use in emergency room situations. The electrode chest pad has upper fit portions with upper limb electrodes, an elongated central base fit portion with a plurality of precordal unipolar electrodes and lower fit portions with lower limb electrodes. The electrodes are attached to leads which are internal to the base chest pad and terminate into a lead branch adapted to plug into an ECG monitor. The base pad material is configured such that one group of electrodes may be separated from a second group of electrodes.

Yet, the configuration and shape of each of the electrode sensor array apparatus described in US 8,868,152 B2, the precordial pad described in US 7,286,865 B2, and the electrode chest pad described in US 6,847,836 B1 is relatively complicated. Also, the application of these configurations to a patient can be time-consuming and complex.

Document US 5,191,886 A1 relates to an electrode strip for use in electrocardiography. The electrode strip comprises a flexible and inextendible substrate, a plurality of conductive leads and an insulating cover layer including a plurality of apertures therethrough. The conductive leads extend from a connector to different ones of the apertures to form electrode sites. Document US 2016/228691 A1 discloses an electrode pad comprising at least one electrode with an electrode terminal. A contact member is disposed on said electrode terminal and covered by a retainer mesh. The electrode pad can comprise an array of several electrodes disposed on a carrier, wherein the carrier has a slit separating at least two neighboring electrodes.

Document US 2013/282814 A1 discloses another electrode patch.

However, the electrode strip of US 5,191,886 A1 and the electrode pad according to US 2016/228691 A1 have a limited number of potential use cases as the positions to which electrodes can be applied are limited.

### OBJECT OF THE INVENTION

It is an object of the present invention to provide an electrode patch, a system comprising an electrode patch, a method of manufacturing an electrode patch and a method of obtaining biometric parameters, which overcome the above drawbacks.

It is a further object of the present invention to provide an electrode patch that is easily and quickly attachable to a subject so as to easily apply and correctly position multiple electrodes to the subject.

It is still another object of the present invention to provide an electrode patch that is suitable for a long-term use and that can be used in multiple clinical setting.

These objects are achieved by the subject matter of the independent claims.

### SUMMARY OF THE INVENTION

According to a first aspect, the present invention provides an electrode patch, in particular for performing electrocardiography, electroencephalography, electromyography, electrooculography (EOG) on a subject and/or for providing electric impulses to a subject either by defibrillation or microdefibrillation. The electrode patch comprises an elastic adhesive layer having an adhesive contact surface that is configured to adhere to the skin of the subject the electrode patch is applied to, an elastic conductive layer including a plurality of measurement points and conductive paths, the conductive paths being configured to conductively connect the plurality of measurement points to a connector, and a plurality of electrodes, wherein each of the plurality of electrodes is formed at least by one of the plurality of measurement points and a corresponding recess provided in the adhesive layer. Each recess extends through the entire layer thickness of the adhesive layer and is provided or providable with a substance that is adhesive and conductive, such as hydrogel, so as to conductively connect the one of the plurality of measurement points to the skin of the subject. At least 90% of the area of the electrode patch is radiolucent by means of radiolucency of the adhesive layer and radiolucency of the conductive layer. Preferably, at least 95% of the area of the electrode patch is radiolucent, more preferably at least 97%. Still more preferably 100% of the area, i.e. the complete area, of the electrode patch is radiolucent. As the electrode patch has a substantially flat shape or configuration, the area of the electrode patch refers to a surface area of the patch.

The term radiolucent as used herein refers to materials or composite materials, which are substantially or fully transparent to electromagnetic, magnetic and/or electric field and/or radiation employed in common (medical) imaging procedures, such as X-ray or MRI, in a manner and degree similar to human soft tissue, e.g. muscle tissue. In particular, the electrode patch can be radiolucent to electromagnetic, magnetic and/or electric field and/or to electromagnetic, magnetic and/or electric field radiation. In other words, the electrode patch according to the invention does not block electromagnetic radiation of X-ray or MRI but allows it to pass, thereby not interferingly emerging in X-ray or MRI pictures. Thus, the electrode patch according to the invention can be worn by a patient during X-ray and/or MRI treatment without negatively affecting the treatment. In particular, radiolucency can mean that the electrode patch, i.e. the radiolucent material, does not darken an image of a common hospital X-ray (RTG) and/or of fluoroscopy and X-ray films taken during angiographies and/or other cardio/neuro/radiological procedures (diagnostic and/or therapeutic) more than 60% of image intensity. An exemplary system for common hospital X-ray can be Siemens Artis Zee, with exemplary settings of the lamp voltage from 40 kV to 70kV and lamp current from 8mA to 12 mA. This maximum darkening should not be exceeded so that the practitioner is still able to assess and evaluate the image. In particular, the radiolucency can mean that the electrode patch, i.e. the radiolucent material, does not darken an image of a common hospital X-ray (RTG) more than 50% of image intensity, preferably not more than 40%, more preferably not more than 30%, still more preferably not more than 28%, still more preferably not more than 18%, still more preferably not more than 5%. The less the image intensity is darkened by the electrode patch, the better the obtained image can be assessed and evaluated by the practitioner. In other words, radiolucency preferably means that the electrode patch, i.e. the radiolucent material, does not attenuate the radiation more than 40.0 µGy/min, preferably not more than 30.0 µGy/min, more preferably not more than 26.0 µGy/ min, still more preferably not more than 23.0 µGy/min. In other words, radiolucency preferably means that during a procedure the ratio of dose of radiation attenuated by the electrode patch, i.e. the radiolucent material, to overall dose of radiation radiated does not exceed 5%, preferably does not exceed 3.5 %, more preferably does not exceed 2%, still more preferably does not exceed 1.7%, still more preferably does not exceed 1.5%.

It will be understood that the exact radiolucency may be different in different regions of the electrode patch, depending, e.g., on the number, thickness and type of layers present in the particular region. For example, the adhesive layer's radiolucency can be greater than the conductive layer's radiolucency.

In contrast to the present invention, prior art medical electrode systems usually comprise metallic components such as metallic leads and cables, connector clips and further radiopaque components. Thus known electrode patches are typically substantially radiopaque and thus show on medical images such as X-ray photographs. In other words, unless the electrodes are taken off prior to an X-ray scan, they will block the passage of X-rays and thus increase the risk of potentially hiding important areas of the X-rayed part of the patient's body on the resulting X-ray picture. In case when the x-ray is used as a visual mean for invasive procedures the electrodes may cause inability for the operator to see certain anatomies or not being able to use certain projections. A long-term use is thus often not possible with conventional electrode patches.

In particular embodiments, each of the layers described herein can be single or multilayered. Further, a layer as described herein can be continuous, such as a closed film, coating, etc., or can be non-continuous, such as a discontinuous structure, pattern, film, coating, etc. that is only provided in some areas of a plane. Hence, it is apparent that the layers of the electrode patch according to the invention, which layers are described above and hereinafter, are not necessarily present in all regions of the electrode patch. For example, some of the layers can only be provided in the region of the patch where an electrode is formed. Yet, there can be regions of the electrode patch, where all of the defined layers are provided.

The substance can be adhesive and is conductive, e.g. hydrogel, spring, foam, chloride layers, can on the one hand isolate and thus protect the subject from the potentially cytotoxic conductive layer and on the other hand provide a conductive connection between the measurement point and the subject. The substance can be a component of the electrode patch according to the present invention. In this case, the substance can preferably be pre-inserted into the recess during the manufacturing process. Alternatively, the substance can be inserted into the recess by the user of the electrode patch.

Elasticity of the electrode patch or the individual patch layers, i.e. stretchability of the electrode patch or the individual patch layers, reduces negative motion artefacts and improves the positioning on the subject.

The elastic adhesive layer can be longitudinally stretchable in a range from 1% to 1000%, preferably from 100% to 900%, more preferably from 200% to 800%, still more preferably from 300% to 500%, still more preferably from 350% to 450%. The elastic conductive layer can be longitudinally stretchable in a range from 1% to 1000%, preferably from 100% to 900%, more preferably from 200% to 800%, still more preferably from 300% to 500%, still more preferably from 350% to 450%. The elasticity of both the adhesive layer and the conductive layer allow for an optimal application and adaption to the subject's shape and dimensions, for example to a patient's body. Thus, the electrode patch is usable for different subject sizes and shapes. In an embodiment, the adhesive layer can have an E-modulus from 1.0 to 600.0 N/mm², preferably from 2.0 to 500.0 N/mm², more preferably from 3.0 to 250.0 N/mm², still more preferably from 3.0 to 20.0 N/mm². The conductive layer can have an E-modulus from 1.0 to 100.0 N/mm², preferably from 2.0 to 50.0 N/mm², more preferably from 3.0 to 25 N/mm², still more preferably from 5.0 to 15.0 N/mm². The E-modulus of the adhesive layer can be greater than the E-modulus of the conductive layer, in particular the ratio between the adhesive layer's E-modulus and the conductive layer's E-modulus can be in the range from 0.6 to 10.0, preferably in the range from 0.6 to 6.0 or 1.0 to 6.0, more preferably in the range from 0.6 to 1.3, still more preferably in the range from 1.0 to 1.3.

The electrode patch can be biocompatible and can be skin friendly so as to avoid skin irritation and enhance the comfortability of the electrode patch. In particular, the adhesive layer can be biocompatible and skin friendly. Preferably, the ratio of cell survival of cells in contact with the electrode patch, in particular with the adhesive layer, during cytotoxicity test can be greater than 0.7, preferably greater than 0.8, more preferably greater than 0.9, tested in line with PN EN ISO 10933-5:2009. The layer structure and/or the fluidic substance can have a cumulative irritation index in the range from 0 to 1.9, more preferably from 0 to 0.4, more preferably from 0 to 0.1, tested in line with PN-EN ISO 10993-10:2015-2. The layer structure and/or the fluidic substance can have a sensitizing effect (grade) in the range from 0 to 1, more preferably of 0, according to the ISO scale defined in PN-EN ISO 10993-10:2015-2.

In an embodiment, the adhesive layer can be at least partially optically transparent. Thus, when the electrode patch is applied to the subject, the adhesive layer does not hinder the viewing and checking of subjacent subject regions. Preferably, the adhesive layer can have light transmission from 15% to 90%, preferably 40% to 90%, for visible light throughout a wave length range between 370 nm to 700 nm. More preferably, the light transmission of the adhesive layer for light with a wave length of 680 nm is substantially similar (such as +/- 10%) to the light transmission for light with a wave length of 550 nm. The refractive index of the adhesive layer can be in the range of 1.0 to 2.5, preferably 1.3 to 1.8, more preferably in the range of 1.4 to 1.6, still more preferably about 1.5.

Also, the conductive layer can be at least partially optically transparent so as to not hinder the viewing and checking of subjacent subject regions. To realize an optically transparent conductive layer, the conductive layer can comprise conductive polymers alone or embedded in a polymer resin.

In an embodiment, the adhesive layer can be double sided adhesive tape, preferably comprising a non-woven layer being arranged between the adhesive contact surface, such as a skin adhesive film, and an adhesive patch surface, such as a patch adhesive film. Alternatively or additionally, the adhesive layer can be at least one of a printed adhesive, a coated adhesive, a dispensed adhesive, a sprayed adhesive, a hot-melted adhesive, a transferable mass and a one-sided tape.

The adhesive layer can comprise acryls, silicones, hydrogels, hydrocolloids, synthetic based rubbers and/or natural rubbers. The material and peel force used for the adhesive layer is chosen depending on whether the subject is to wear the electrode patch for only a couple of seconds, i.e. achieving a sufficiently simple detaching of the patch, or for a couple of days or even weeks, i.e. ensuring a sufficient adhesive strength even though the subject may be sweating during a long-term use. In particular, the adhesive contact surface of the adhesive layer can have a peel force from 0.5 to 50.0 N/25mm, preferably from 10.0 to 40.0 N/25mm, more preferably from 20.0 to 40.0 N/25mm, still more preferably from 30.0 to 36N/mm. For example, a peel force of less than 5.0 N/25mm, preferably of 1.0 N/25mm can be chosen if the electrode patch is only to be worn for a really short term, such as less than 2 hours. For example, a peel force of more than 30 N/mm, e.g. 36N/25mm or even 40N/25mm, can be chosen if the electrode patch is to be worn for at least 10, preferably at least 14 days. As a testing method for determining the peel force, testing 180° peel on Bristol paper can be used.

Besides the adhesive contact surface, the adhesive layer can also have an adhesive patch surface, which opposes the adhesive contact surface that is oriented towards the subject, when the patch is used in a predetermined manner. The adhesive patch surface is configured to adhere to one or more adjacent layer/s of the electrode patch. The adhesive patch surface can have a peel force from 0.5 to 500.0 N/25mm, preferably from 10.0 to 200.0 N/25mm, more preferably from 20.0 to 100.0 N/25mm, still more preferably from 30.0 to 50 N/mm. As a testing method for determining the peel force, testing 180° peel on Bristol paper can be used. The peel force of the adhesive patch surface can be the same or greater than the peel force of the adhesive contact surface.

Moreover, the adhesive layer can have a density in the range from 0.7 to 1.2 g/cm³, preferably from 0.8 to 1.1 g/cm³, more preferably from 0.9 to 1.0 g/cm³, at approx. 23°C. Furthermore, the adhesive layer can have a penetration value from 150 to 270 mm/10, preferably from 180 to 240 mm/10, more preferably from 200 to 220 mm/10, still more preferably from approx. 210 mm/10, tested with a hollow cone of 62.5 g according to DIN ISO 2137:2007.

Apart from the adhesive layer, some or all of the other layers of the patch can be bonded adhesive-free, in particular by lamination, thermal bonding or ultrasonic bonding. In particular, the carrier layer and the support layer can be laminated.

In an embodiment of the electrode patch, the conductive layer comprises a conductive particle composition, preferably having an elastic polymer resin, such as a polyurethane, a silicone, a rubber and/or a polydimethylsiloxane resin. In particular, the conductive particle composition can be a silver-carbon paste. Preferably, the silver-carbon paste has at least 50 wt% silver paste so as to ensure a sufficient conductivity. On the other hand, the amount of silver paste of the silver-carbon paste can be reduced, e.g. to less than 90 wt%, preferably less than 80 wt%, so as to reduce costs. On the other hand to maintain the paste's conductivity even under stretching best possibly, the amount of carbon paste can be increased, which will be described in more detail hereinafter. The silver-carbon paste can have 50 wt% to 90 wt% silver paste, more preferably 60 wt% to 80 wt% silver paste, still more preferably 70 wt% silver paste. The silver paste can have 30 wt% to 80 wt% solid constituents, preferably 40 wt% to 70 wt%, more preferably 50 wt% to 60 wt%. The solid constituents can be the conductive particles of the composition and can have the form of at least one of flakes, powders, particles, micro-particles, nano-particles, nano-tubes, spheres, nano-wires, micro-wires, wires and others. The weight-percentage of the silver-carbon paste that is not silver paste can be carbon paste only or can be carbon paste in addition to other components.

It will be understood that the conductive particle composition can be provided by mixing at least two pastes with different functional phases, such as silver paste and carbon paste, each comprising a polymer resin, wherein the resins have to be compatible. Alternatively, the conductive particle composition can be provided by one paste having at least one functional phase, such as carbon paste only or silver paste only, or by one paste having at least two different functional phases. Alternatively, the conductive particle composition can be provided by one paste having at least one conductive phase, for example conductive polymer paste.

Even though, silver and carbon have been mentioned above as exemplary conductive components, principally all conductive materials can be used instead of or in addition to silver and/or carbon, such as Au, Cu and other metals, conductive polymers and/or hydrogels.

To ensure sufficient conductivity, the conductive layer should have a maximum resistivity of 0.005 Ωm for frequencies from DC to 200 kHz. The resistivity of the conductive layer is preferably less than 0.003 Ωm, more preferably less than 0.0005 Ωm, still more preferably less than 0.00001 Ωm, still more preferably less than 0.000001 Ωm for frequencies from DC to 200 kHz.

As the conductive layer is elastic, i.e. stretchable, the conductive layer has to maintain its conductivity sufficiently even under stretching. In a preferred embodiment, a conductive path having a width of 4 mm, when longitudinally elongated to a length of 150% of its original length, can still have a resistivity of less than 0.5 Ωm, preferably less than 0.1 Ωm, more preferably less than 0.05 Ωm, still more preferably less than 0.03 Ωm, in particular while when longitudinally elongated to a length of 175% of its original length, has still a resistivity of less than 1.0 Ωm, preferably less than 0.75 Ωm, more preferably less than 0.5 Ωm, still more preferably less than 0.3 Ωm, and in particular while when longitudinally elongated to a length of 200% of its original length, has still a resistivity of less than 10 Ωm, preferably less than 5 Ωm, more preferably less than 2.5 Ωm, still more preferably less than 1.7 Ωm.

Further, the conductive path having a width of 2 mm, when longitudinally elongated to a length of 150% of its original length, can still have a resistivity of less than 0.5 Ωm, preferably less than 0.25 Ωm, more preferably less than 0.1 Ωm, still more preferably less than 0.05 Ωm, in particular while when longitudinally elongated to a length of 175% of its original length, has still a resistivity of less than 1.0 Ωm, preferably less than 0.75 Ωm, more preferably less than 0.5 Ωm, still more preferably less than 0.3 Ωm, and in particular while when longitudinally elongated to a length of 200% of its original length, has still a resistivity of less than 15 Ωm, preferably less than 10 Ωm, more preferably less than 8 Ωm, still more preferably less than 7.5 Ωm, still more preferably less than 5 Ωm.

Further, the conductive path having a width of 4 mm, when longitudinally elongated to a length of 150% of its original length, preferably does not increase its resistivity more than 10000%, more preferably not more than 7000%, still more preferably not more than 5000%, still more preferably not more than 4000%.

Further, the conductive path having a width of 2 mm, when longitudinally elongated to a length of 150% of its original length, preferably does not increase its resistivity more than 12000%, more preferably not more than 10000%, still more preferably not more than 8000%, still more preferably not more than 6000%.

Referring to the silver-carbon paste, it has been observed that a silver-carbon paste with a higher amount of carbon is more resistant for stretching in view of its conductivity, i.e. such a paste has a lower increase of resistivity when stretched.

Moreover, the radiolucency of the conductive layer can be adjustable by varying or selecting the conductive particle composition. Regarding the exemplary silver-carbon paste, a higher amount of carbon paste leads to a higher radiolucency of the silver-carbon paste. However, even a conductive layer consisting completely of silver paste can be sufficiently radiolucent when, e.g., polymer resin comprising conductive silver particles, wherein the conductive layer is relatively thin. For example, thin can describe a thickness of at least less than 100 um, preferably less than 5 um, more preferably less than 25 um. Such a thin conductive layer can be realized by providing a respective composite deposition.

In an embodiment, selected portions of the conductive layer, such as one or more measurement points, can be covered by one or multiple cover layer/s. The cover layer can be provided in the recess, e.g. in addition or alternative to the substance or can be the substance The cover layer can be disposed between the conductive layer and the surface of the subject the electrode patch is applied to, preferably between a measurement point of the conductive layer and the corresponding adhesive and conductive substance. For example, the cover layer can be provided by chlorinating the conductive layer in a predetermined or selected portion or by providing a separate layer, such as a silver chloride layer. The cover layer can improve the interface between ionic current on the subject's skin and electron current in the conductive layer. Further, the cover layer can prevent contact of the subject with the conductive layer, which can e.g. contain cytotoxic components such as silver. With other words, the cover layer can provide an additional isolation between the measurement point/s and the subject, for example in addition to adhesive and conductive substance. It will be understood that apart from chloride or silver chloride also other cover layers are possible.

In an embodiment, the electrode patch can further comprise a carrier layer, wherein the conductive layer is arranged between the carrier layer and the adhesive layer. Further, the carrier layer is also radiolucent, preferably substantially fully translucent to X-ray and MRI radiation. The carrier layer can provide stability to the electrode patch. In an alternative embodiment without a carrier layer, just the conductive layer is applied to the subject by means of the adhesive layer.

The carrier layer and/or the adhesive layer are preferably a closed or continuous layers that substantially resemble or form the electrode patch surfaces. The carrier layer can form the electrode surface facing away from the subject and the adhesive layer forms the electrode surface facing towards the subject, when the patch is applied in the predetermined manner to the subject.

The carrier layer can be elastic and preferably has a longitudinal stretchability in a range from 1% to 1000%, preferably from 100% to 900%, more preferably from 200% to 800%, still more preferably from 300% to 500%, still more preferably from 350% to 450%. The elastic carrier layer can have an E-modulus from 8.0 to 20.0 N/mm², preferably from 8.0 to 15.0 N/mm², more preferably from 8.0 to 13.0 N/mm², still more preferably from 9.0 to 12.0 N/mm², still more preferably from 10.0 to 11.0 N/mm². In a preferred embodiment, the ratio between the longitudinal elongation of the adhesive layer at maximum stress and the longitudinal elongation of the carrier layer at maximum stress is greater than 0.5 but less than 1.0 so that the adhesive layer does not limit the mechanical properties of the electrode patch set by the carrier layer. Preferably, the aforementioned ratio is greater than 0.7 but less than 1.0, more preferably greater than 0.8 but less than 1.0, still more preferably greater than 0.9 but less than 1.0.

Further, the carrier layer can be a polymer layer, such as a thermoplastic polymer layer, in particular at least one of a TPU layer, a PET layer, a silicone layer, etc. Alternatively or additionally, the carrier layer can comprise other materials such as papers or textiles. The carrier layer can be non-woven, woven, a film, a foam, a coating, etc.

Also, the polymer layer can be siliconized. This can for example be achieved by casting the polymer resin onto a siliconized paper during manufacturing. Such paper can also determine the roughness of the carrier layer. Exemplary roughness (tested according to ISO 4287:1999) of the carrier layer comprised in the electrode patch may be in the range from 1 to 1.5 µm Pa, 1.2 to 1.7 µm Pq, in the range from 6.4 to 6.9 µm Pz and 3.6 to 4.1 Pp.

The thickness of the carrier layer can be from 30 to 70 µm, preferably from 40 to 60 µm, more preferably about 50 µm. Such layer thicknesses allow to provide an optimal balance between comfortability, stability and stiffness, while being thin and stretchable. Comfortability helps to reduce skin irritation, whereas stability and stiffness supports the applicability of the patch to the subject. To ensure comfortability and further reduce skin irritation, thus increasing the term for which the electrode patch can be worn and enabling long term use, the electrode patch has to be soft particularly in the regions of the patch's edges. Therefore, the carrier layer can have a hardness (5 sec) of 80 to 105, preferably 85 to 100, more preferably 89 to 95, still more preferably about 92, Shore A. Furthermore, the material of the carrier layer can be breathable. Moreover, the carrier layer can be water-proof.

In a preferred embodiment, the carrier layer can be biodegradable, which contributes to ecofriendliness.

It is to be mentioned that even if the carrier layer is not comprised in the assembled electrode patch that is applied to the subject, the carrier layer can be provided during manufacturing of the electrode patch to carry electrode patch layers during the manufacturing process so as to support the manufacturing of the patch. This will be described in more detail further below.

In an embodiment, the carrier layer can be optically transparent. Preferably, the carrier layer can have light transmission from 15% to 90% for visible light with a wave length between 370 nm to 700 nm. More preferably, the light transmission of the carrier layer for light with a wave length of 680 nm is greater than the light transmission for light with a wave length of 550 nm. Wherein in particular, the light transmission of the carrier layer for light with a wave length of 680 nm is 1% to 10% greater than the light transmission for light with a wave length of 550 nm (i.e. percentage light transmission for 680 nm = percentage light transmission for 550 nm plus 1% to 10%), preferably 2% to 5% greater, more preferably 2 to 3% greater. Preferably, for light with a wave length of 680 nm the light transmission of the carrier layer is from 70% to 90%, still more preferably from 75% to 80%, still more preferably from 77% to 79%. Thus, when the electrode patch is applied to the subject, the carrier layer does not hinder the viewing and checking of subjacent subject regions. In this context, it is further mentioned that the refractive index of the carrier layer can be in the range of 1.0 to 2.5, preferably 1.3 to 1.8, more preferably in the range of 1.4 to 1.6, still more preferably in the range of 1.49 to 1.57.

In an embodiment, the electrode patch can further comprise a description layer visualizing at least one of the plurality of measurement points and/or at least one instruction and/or at least one reference point for supporting the predetermined positioning of the electrode patch on the subject. The description layer can be disposed on the carrier layer, i.e. between the carrier layer and the conductive layer. Preferably, the description layer or the description and conductive layer is/are the only layer/layers in the electrode patch that are not transparent. Consequently, when applying the electrode patch to the subject, the user is able to see the description layer through the carrier layer and can see the subject through the carrier layer and the adhesive layer (and preferably the conductive layer) and can thus match portions of the description layer with predetermined marks, reference points, anatomic features, etc. For example, intercostal and sternal lines can be used as reference points, thus preventing the common mistake of placing V1/V2 electrodes at the second intercostal space instead of at the fourth intercostal space. Thus, the positioning is simplified and the electrode patch can be positioned more precisely and more quickly. The improved positioning also enhances the reproducibility and consistency of measurements conducted with the electrode patch. Preferably, at least 80% of the electrode patch (referring to its surface area) can be at least 15% to 90% optically transparent, preferably at least 40% optically transparent, i.e. can have light transmission from 15% to 90%, preferably at least 40%, for visible light with a wave length between 370 nm to 700 nm.

In an embodiment, the electrode patch can further comprise a dielectric layer being arranged between the adhesive layer and the conductive layer, wherein the recess also extends through the entire layer thickness of the dielectric layer. In particular, the dielectric layer can be provided at least in the regions of the conductive layer, so as to cover and insulate at least the conductive paths and the parts of the measurement points that extend beyond the cross-section area of the recess, i.e. the parts of the measurement points that do not overlap the cross-section area of the recess. Thus, the dielectric layer ensures that in case the adhesive layer is conductive current can only be transmitted between the subject and the conductive layer via the recesses. Here, dielectric means that the dielectric layer insulates the conductive layer at least from or towards the subject.

Additionally or alternatively, the adhesive layer itself can be dielectric. In particular, the adhesive layer can be dielectric at least in the regions of the conductive layer, so as to insulate at least the conductive paths and the parts of the measurement points that extend beyond the cross-section area of the recess, i.e. the parts of the measurement points that do not overlap the cross-section area of the recess. Thus, the dielectric adhesive layer ensures that current can only be transmitted between the subject and the conductive layer via the recesses.

The dielectric, i.e. the dielectric layer and/or the dielectric adhesive layer, can have at least 10 GΩ insulation resistance, preferably at least 15 GΩ, more preferably at least 18 GΩ, still more preferably at least 20 GΩ, still more preferably at least 50 GΩ, still more preferably at least 100 GΩ. The breakdown voltage for the dielectric can be at least 100 V, preferably at least 500 V, more preferably at least 1 kV, still more preferably at least 2 kV, still more preferably at least 2.5 kV, still more preferably at least 4 kV.

Further, the layer combination of the carrier layer, the dielectric layer and the adhesive layer can have light transmission from 15% to 90% for visible light with a wave length between 370 nm to 700 nm, more preferably from 30% to 70%, still more preferably from 40% to 55%. More preferably, the light transmission of such a layer combination for light with a wave length of 680 nm is greater than the light transmission for light with a wave length of 550 nm. Wherein in particular, the light transmission of such a layer combination for light with a wave length of 680 nm is 1% to 15% greater than the light transmission for light with a wave length of 550 nm (i.e. percentage light transmission for 680 nm = percentage light transmission for 550 nm plus 1% to 15%), preferably 3% to 10% greater, more preferably 5 to 8% greater. Preferably, for light with a wave length of 680 nm the light transmission of such a layer combination is from 20% to 90%, still more preferably from 30% to 80%, still more preferably from 40% to 60%, still more preferably from 45% to 55%.The dielectric layer can have a refractive index in the range of 0.7 to 2.5, preferably 1.0 to 1.8, more preferably in the range of 1.2 to 1.6, still more preferably about 1.4.

In an embodiment, the electrode patch is partly perforated. The perforation increases the breathability of the electrode patch, reduces skin irritations and thus supports a long-term use of the electrode patch. To achieve a sufficient breathability from 20% to 90% of the electrode patch surface area can be perforated, i.e. can comprise perforations, preferably at least 40%, more preferably at least 50%, still more preferably at least 70%. In particular, apart from the conductive layer, all layers of the electrode patch can be at least partly perforated. The cut out surface area formed by the perforations can be 20% to 60% of the total electrode patch surface area, preferably 30% to 50%, more preferably about 40%. Each perforation can have a diameter between 0.01 mm and 5.0 mm, preferably between 0.025 mm and 2.5 mm, more preferably from 0.05 mm to 1.0 mm, still more preferably from 0.1 to 0.5 mm. The perforation can have a pitch between 0.1 and 0.2 mm, preferably about 0.15 mm. Further, at least 30% of the

The electrode patch can have a thickness between 10 µm and 1000 µm in the area of the at least one measurement point and a thickness between 10 µm and 1000 µm in the other areas. Preferably, the thickness in the area of the at least one measurement point is larger than the thickness in the other areas of the patch. A reason can be that some layers and/or components are preferably only provided in the area of the measurement points, such as the dielectric layer or the adhesive and conductive substance. The thickness in the area of the at least one measurement point can be at least 100 µm, preferably at least 500 µm, more preferably at least 750 µm and still more preferably at least 900 µm. The thickness in the other areas of the patch can be between 20 µm and 500 µm, preferably between 40 µm and 300 µm, more preferably between 50 µm and 250 µm, still more preferably between 70 µm and 190 µm. It is advantageous regarding the wearing comfortability if the electrode patch is possibly thin, while achieving all of the above described effects in particular in the areas of the measurement points.

As described above, the carrier layer can have a preferred thickness of about 50 µm. The ratio between the thickness of the description layer and the thickness of the carrier layer can be from 0.1 to 0.7, preferably from 0.3 to 0.5. The ratio between the thickness of the conductive layer and the thickness of the carrier layer can be from 0.1 to 0.7, preferably from 0.3 to 0.5. The ratio between the thickness of the dielectric layer and the thickness of the carrier layer can be from 0.1 to 0.8, preferably from 0.4 to 0.6. The ratio between the thickness of the adhesive layer and the thickness of the carrier layer can be from 0.2 to 0.9, preferably from 0.5 to 0.7. The ratio between the thickness of the description layer and the thickness of the conductive layer can be from 0.5 to 2.0, preferably from 1.0 to 1.5. The ratio between the thickness of the conductive layer and the thickness of the dielectric layer can be from 0.1 to 0.9, preferably from 0.7 to 0.8, preferably from 0.5 to 0.7 The ratio between the thickness of the dielectric layer and the thickness of the adhesive layer can be from 1.0 to 0.5, preferably from 0.8 to 0.7. The ratio between the thickness of the adhesive and conductive substance and the thickness of the conductive layer can be from 1.0 to 200.0, preferably from 20 to 100.

The E-modulus of the electrode patch comprising a combination of layers including an adhesive layer, a conductive layer, a dielectric layer and a carrier layer can have an E-modulus in the range from 2.0 N/mm² to 100 N/mm², preferably from 3.0 N/mm² to 50 N/mm², more preferably from 4.0 N/mm² to 15 N/mm², still more preferably from 5.5 N/mm² to 8.0 N/mm².

The electrode patch comprises a reinforcing adhesive layer, which is provided in predetermined positions or areas of the patch so as to locally reinforce the adhesiveness of the electrode patch, more precisely to reinforce the adhesiveness of the adhesive layer. In particular, the reinforcing adhesive layer can be arranged in an area of or adjacent to the edges of the electrode patch, in an area around each electrode (e.g. in the form of rings) and/or in an area in which the connector or device is attachable. With other words, the electrode patch comprises a hybrid layer comprising the adhesive layer and the reinforcing adhesive layer. A ratio of the surface area of the adhesive layer compared to the surface area of the reinforcing adhesive layer can be at least 5/2, preferably at least 4/1, more preferably at least 5/1, still more preferably at least 6/1. The peel force of the reinforcing adhesive layer can be greater than the peel force of the adhesive layer. The adhesive layer can comprise or be made of a silicone adhesive, wherein the reinforcing adhesive layer can comprise or be made of an acrylic adhesive. Thus, by providing an adhesive layer and a reinforcing adhesive layer, the overall peel force of the patch to the subject can be increased, while still guaranteeing sufficient biocompatibility of the patch.

In an embodiment, the electrode patch can further comprise a support layer, which can be applied to the electrode patch adjacent to the carrier layer on a surface of the carrier layer opposing the surface that faces the conductive and adhesive layer. The support layer can provide support and stability to the electrode patch during manufacturing and can be removed after manufacturing. The support layer can be a polymer layer, such as a siliconized PET layer. The support layer can be stretchable, which improves the handling during manufacturing.

In an embodiment, the electrode patch can further comprise an outer cover layer, such as a backing foil, which removably adheres to the adhesive layer so as to cover and protect the adhesive layer during transportation. The outer cover layer can be removed from the adhesive prior to application to the subject.

The electrode patch can comprise at least 2 electrodes, preferably at least 12 electrodes, more preferably at least 15 electrodes, still more preferably at least 18 electrodes. The electrode patch can for example comprise from 2 to 100 electrodes. The electrode patch can comprise more than 100 electrodes. Thus, the electrode patch can for example be used for a multiple-lead ECG. In particular, the electrode patch can comprise 2 to 18 electrode, preferably 12, 15 or 18 electrodes. Each electrode can be formed as specified above. Thus, the number of measurement points and recesses can correspond to the number of electrodes comprised in the electrode patch. For example, the electrode patch can bolster a simultaneous reading for 18-lead ECG.

In an embodiment, the electrode patch can have a single-piece form. Such a single-piece form improves the handling of the electrode patch and its application to the subject. Compared to prior art patches, a single-piece electrode patch does not have to be assembled prior to or during applying and positioning and thus reduces the time needed for positioning. The electrode patch can comprise a plurality of electrode patches that are mechanically and electrically connected by thermal bonding or laminating.

The electrode patch can comprise a first portion which is designed to extend to the right side of the subject's sternal midline in an attached state of the electrode patch, a second portion which is designed to extend to the left side of the subject's sternal midline in an attached state of the electrode patch, and a third portion which is designed to being disposed posteriorly in an attached state of the electrode patch. Such a design is particularly advantageous regarding a quick and correct positioning as will be described below in more detail. This configuration provides electrodes embedded in the electrode patch that also record posterior leads, which results in higher accuracy and an increase in the number of leads to visualize ST segment changes and to diagnose a heart attack in the posterior wall or occluded right sided arteries that currently are difficult to diagnose. Further, for patients with dextrocardia the afore-described configuration can be mirrored. Also, the shape and outline of the electrode patch can be adapted specifically for males and females as in guidelines, electrodes for females must go beneath the breast in contrast to electrodes for males.

Each of the first portion and the second portion of the electrode patch can comprise at least three aligned electrodes, which are aligned along a straight line, respectively. Each of the straight lines can be substantially parallel to the sternal midline of a patient in a state in which the electrode patch is attached to the patient in a predetermined manner. The aligned electrodes can be provided to obtain limb leads during ECG. Therefore, the aligned electrodes can be spaced approx. 5 cm to 25 cm, preferably 10 cm to 20 cm, more preferably 12.5 cm to 17.5 cm, still more preferably about 15 cm, from a region or line where the first portion and the second portion of the electrode patch are connected.

In another embodiment, the electrode patch comprises the connector, which is conductively connected to the conductive paths and is conductively connected or connectable to an internal or external device so that signals can be transferred from the conductive paths to the device via the connector. The connector can be a reversing plate. The connector can be a polymer based connector that is radiolucent. The connector can comprise the same material as the carrier layer. Thus, it doesn't require removal of electrode for X-ray, MRI or CT examination. Further, the connector can be optically transparent. The connector can be a single socket connector. In some preferred embodiments, the connector can be configured to bare the conductive paths in a direction facing away from the subject in an attached state of the electrode patch. This applies in particular for an embodiment in which the patch comprises a carrier layer that covers the conductive layer towards the patch surface facing away from the subject in an attached state of the patch. The connector can be a polymer plate having conductive traces on its surface that faces away from the subject in an attached state of the patch. The connector or related parts can be crimped, mounted with an adhesive, ultrasonically or thermally bonded, i.e. laminated, to the other patch layers. The connector can be manufactured by scree-printing.

The connector can be mounted to the electrode patch so that its conductive traces face and partially overlap the conductive paths of the conductive layer, more precisely one conductive trace of the connector partially overlaps only one corresponding conductive path of the conductive layer, thereby reversing the facing direction of the conductive paths from towards the subject to a direction away from the subject in an attached state of the patch. The conductive traces of the connector can have the same composition as the conductive layer. The electrode patch can comprise a recess or gap, wherein the connector is arranged substantially in the region of the recess or gap so that the connector and the connector and the other patch structure (e.g. the adhesive layer, the conductive layer, the carrier layer) only partially overlap so as to conductively connect the conductive traces and the conductive paths.

By realizing all of the electrode patches conductive connections via the conductive layer, the substance and the conductive traces of the connector, the electrode patch can be entirely cable-free, which increases the comfort for the subject the patch is applied to. Further, it ensures that electrodes do not fall off inadvertently. Moreover, less cables lead to less electromagnetic interference harvesting.

In a further embodiment, the device is magnetically and/or mechanically, removably connectable to the connector. The connector can be provided with one or more magnets, a snap connector, an adhesive, or a clip connector, while the device can be provided with the corresponding counterpart so as to establish a removable connection. Alternatively, the device and the connector can be a single integrated component. Preferably, the device comprises a transmitter so as to wirelessly transfer the signals obtained by means of the electrode patch to a processor or analysis unit. For the wireless transmission radio communication like Bluetooth, near field communication, WLAN, ZigBee, Z-Wave, LoRa and/or GPRS can be used. Alternatively, the device can transmit the signals via cables. The device can be used for an automated method of interpretation of the signals. Also, continuous monitoring can be carried out with live wireless transmission of the obtained signals from the electrode patch to a remote device, such as a smartphone, tablet, laptop, etc.

The exemplary and preferred ranges specified above comprise particular parameter values that are particularly sufficient to achieve and balance all of the discussed technical effects and advantages, such as radiolucency, optical transparency, elasticity and stretchability, wearing comfort, simple and correct positioning, while reducing manufacturing costs. The electrode patch according to the invention can be uses during medical procedures, such as X-ray, CT, MRI or angiography, during physical performance testing, in medical emergencies, such as cardiac arrest and arrhythmias, while performing medical imagine, such as coronary angiography and stent placement, for hospital ECG monitoring, both acute and long-term, at home, for point of care ECG, ischemia diagnosis and monitoring, and others.

According to another aspect, the present invention provides a system, in particular for performing electrocardiography, electroencephalography or electromyography on a subject. The system comprises an electrode patch as described above, a connector which is conductively connected to the conductive paths, and an internal or external device which is conductively connected or connectable to the connector so that signals can be transferred from the conductive paths to an internal or external device via the connector. The connector and/or the device can have the above defined configurations and features.

According to another aspect, the present invention provides a method of manufacturing an electrode patch, in particular an electrode patch as described above. The method comprises the steps: providing an elastic adhesive layer having an adhesive contact surface that is configured to adhere to the skin of a subject the electrode patch is applied to; providing an elastic conductive layer including a plurality of measurement points and conductive paths, the conductive paths being configured to conductively connect the plurality of measurement points to a connector; and embedding a plurality of electrodes in the electrode patch, wherein each of the plurality of electrodes is formed at least by one of the plurality of measurement points and a corresponding recess provided in the adhesive layer, which recess extends through the entire layer thickness of the adhesive layer and is provided or providable with a substance that is adhesive and conductive, such as hydrogel, so as to conductively connect the one of the plurality of measurement points to the skin of the subject; wherein at least 90% of area of the electrode patch is radiolucent by means of radiolucency of the adhesive layer and the conductive layer.

The electrode patch can be manufactured for example by screen printing, stencil printing, inkjet printing, aerosol-jet printing, flexography or other fabrication techniques such as chemical etching, laser ablation, hot embossing, embossing or photoengraving, employing machines that operate either 'sheet-by-sheet', 'roll-to-roll' or combine both. A preferred method uses screen printing.

In the following, examples for preferred steps of the method of manufacturing are described.

In a first step of the method of manufacturing, the carrier layer can be connected to the support layer, e.g. by attaching with an adhesive or by lamination. The support layer can be laminated adhesive-free to the carrier layer. Adhesive-free lamination reduces the risk of patch contamination, e.g. with biotoxic substances. The lamination can be performed with a lamination machine in temperature ranges from 25 °C to 180 °C, preferably from 100 °C to 160 °C, more preferably from 110 °C to 150 °C. The machine can be a roll laminator as well as a sheet laminator. The carrier layer and the support layer are laminated to achieve suitable substrate stiffness needed for subsequent printing. The support layer is chosen so that it is resistant for elevated temperature (e.g. 145 °C) during thermal curing of printing pastes and allows the precise placement of the electrode patch on a vacuum table. The support layer can have a tack force in the range from 75 to 120 g/in (int.method from FTM10 - Tesa Tape 7475). If the tack force is too low it would be impossible to print onto the carrier layer since the carrier would detach from the support layer. On the other hand, if the tack force is too high it would be impossible to later detach the support layer from the electrode patch, more precisely from the carrier layer. The support layer is removed prior to use of the electrode patch to achieve desired properties of the patch as described above.

In a second step of the method of manufacturing, the description layer describing e.g. measurement points and reference points can be printed onto the carrier layer, more precisely onto the surface facing away from the support layer. For example, screen printing with the use of a silk screen with a mesh from 50 to 120 T is possible. Alternatively, it is also possible to print with a steel screen. The description layer can then for example be cured in a chamber dryer for 15 min at 90°C. The descriptions can alternatively be cured in tunnel dryers, photonically cured or UV cured, etc. depending on the type of the material used for printing.

In a third step of the method of manufacturing, the conductive layer can be printed onto the carrier layer, more precisely onto the surface facing away from the support layer, possibly partially overlapping the description layer. The conductive layer can then be cured in e.g. tunnel dryers or photonically cured depending on the type of the conductive layer. The drying can be performed for 15 min at 120°C.

In a fourth step of the method of manufacturing, the dielectric layer can be printed onto the carrier layer, more precisely onto the surface facing away from the support layer, possibly partially overlapping the description layer and the insulatingly overlapping the conductive layer. The dielectric layer can be provided so as to cover either all the electrode patch area except of measurement points with the adhesive and conductive substance or to cover at least the conductive layer except of measurement points with the adhesive and conductive substance. The dielectric layer can be cured e.g. in tunnel dryers, photonically cured or UV cured, etc. depending on the type of the dielectric layer. For example, the dielectric layer can be first dried by means of a UV belt dryer and can subsequently be dried for 15 min at 90°C in a chamber dryer.

In a fifth step of the method of manufacturing, the adhesive layer can be printed, transferred or applied onto the carrier layer, more precisely onto the surface facing away from the support layer and thus onto the description layer, the conductive layer and the dielectric layer. The adhesive layer has recesses in the regions aligning with the measurement points of the conductive layer. Such recesses can be provided either during printing, more precisely by not printing any adhesive in these regions, or before transferring or applying the adhesive layer by cutting respective recesses into the adhesive layer. In case the adhesive layer is printed, it can be dried e.g. for 15 min at 120°C. The adhesive layer can be cured in tunnel dryers, photonically cured or UV cured, etc. depending on the type of the adhesive layer.

In a sixth step of the method of manufacturing, the substance, such as hydrogel, can be inserted into the recesses of the patch, i.e. into predetermined positions. Substance placement can be performed by hand, by transfer from a roll or sheet, or automated by robotic placers like SMD placers or other robots and manipulators (i.a. Delta, SCARA, Cartesian, Cylindrical, Polar, Jointed-arm etc.). Depositing the hydrogel can be performed by a dispensing robot. Liquid hydrogel can be dispensed from the syringe. Prior to inserting the substance, the substance can be die cut or laser cut into the predetermined shape, in other words corresponding to the shape of the recesses.

In a seventh step of the method of manufacturing, the outer cover layer can be applied to the adhesive layer of the electrode patch. Applying the outer cover layer can be performed by hand, by transfer from a roll or sheet, or automated by robotic placers like SMD placers or other robots and manipulators (i.a. Delta, SCARA, Cartesian, Cylindrical, Polar, Jointed-arm etc.).

In an eighth step of the method of manufacturing, the support layer can be removed. It can be peeled off by hand or automated.

In a ninth step of the method of manufacturing, the patch can be perforated. The perforating can be performed by means of a perforating roll covered needles, e.g. covered with from 250 to 1250 needles with length from 0.5 mm to 3.0 mm. Alternatively, the perforation can be performed e.g. by means of die cut, laser cut or drilling. In particular by using laser cutting it can be ensured that the conductive paths are not perforated.

In a tenth step of the method of manufacturing, the outer cover layer can be cut so as to separate the outer cover layer portions that cover the above described first, second and third portions of the electrode patch. The cutting can be done by hand tools like scissors or knives, by laser cutting, by die cutting or by cutting plotters.

In an eleventh step of the method of manufacturing, the outline of the electrode patch can be cut. The cutting can be done by hand tools like scissors or knives, by laser cutting, by die cutting or by cutting plotters.

The afore-described steps can be performed in various order or in the order defined by the above given order. Also, some of the steps can be performed together in a single step and/or simultaneously. For example, the ninth step can be performed prior to the eighth step. The eleventh step can be performed in a single step together with the tenth step.

According to another aspect, the present invention provides a method of obtaining biometric parameters from a subject by use of an electrode patch as described above. In particular, the method can comprise performing electrocardiography, electroencephalography, electromyography, and/or electrooculography (EOG) on a subject and/or for providing electric impulses to a subject either by defibrillation or microdefibrillation, wherein the subject can be a patient.

The method can comprise the step of applying the electrode patch to the subject by separately and successively attaching initially a first portion of the electrode patch, which is designed to extend to the right side of the subject's sternal midline in an attached state of the electrode patch, subsequently a second portion of the electrode patch, which is designed to extend to the left side of the subject's sternal midline in an attached state of the electrode patch, and then a third portion of the electrode patch, which is designed to being disposed posteriorly in an attached state of the electrode patch. The electrode patch according to the invention can be placed fully and correctly in under 20 seconds (with right sided leads and posterior leads). In contrast, conventional electrode patches for a standard 12-lead ECG need roughly 2 minutes to be positioned by a trained person. It is to be mentioned that each of the above steps of attaching the first, second and third portions of the patch can include removing a corresponding first, second and third portion of an outer cover layer of the electrode patch.

Even though, some of the features, functions, embodiments, technical effects and advantages have been described with regard to the electrode patch or with regard to its manufacturing method, it will be understood that these features, functions, embodiments, technical effects and advantages can also apply accordingly to the electrode patch, the system, the method of manufacturing and/or the method of obtaining biometric parameters.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:
Fig. 1 shows a schematic cross sectional view of a first embodiment of the electrode patch, displaying the electrode patch configuration in the region of a measurement point.
Fig. 2 shows a schematic cross sectional view of the first embodiment of the electrode patch, displaying the electrode patch configuration in the region of a conductive path.
Fig. 3 schematically shows an overall shape of an embodiment of the electrode patch in top view.
Fig. 4 shows a schematic cross sectional of the first embodiment of the electrode patch, displaying the electrode patch configuration during manufacturing.
Fig. 5 shows a schematic cross sectional view of a second embodiment of the electrode patch, displaying the electrode patch configuration in the region of a measurement point.
Fig. 6 shows a schematic cross sectional view of an embodiment of the adhesive layer.
Fig. 7 shows a schematic view of a further embodiment of the electrode patch comprising a hybrid adhesive layer.

### DETAILED DESCRIPTION OF THE DRAWINGS

Various examples of embodiments of the present invention will be explained in more detail by virtue of the following embodiments illustrated in the figures and/or described below.

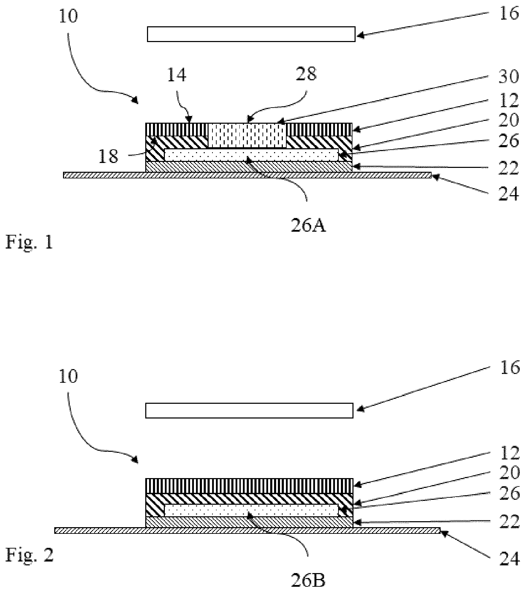

Figure 1 shows the layers of a first embodiment of the electrode patch 10 according to the invention in the region of a measurement point. The electrode patch 10 comprises an adhesive layer 12 having an adhesive contact surface 14 which when the patch is used in the predetermined manner is oriented towards a subject surface 16. The adhesive contact surface 14 is configured to adhere to the subject surface 16, in particular to the skin of a subject such as a patient. The adhesive layer 12 can for example be an adhesive polymer film or coating or a double sided adhesive tape. The adhesive layer 12 is stretchable, which improves the positioning and wearing comfort of the electrode patch.

Further, opposite to the adhesive contact surface 14, the elastic adhesive layer 12 has an adhesive patch surface 18 that is configured to adhere to adjacent surfaces. As shown in the embodiment of Figure 1, in the region of the measurement point, the adjacent surface is a surface of a dielectric layer 20. It will be understood that in other regions of the patch, the adhesive layer 12 can be in contact with other layers than the dielectric layer 20, e.g. with a description layer 22 and/or with a carrier layer 24, since the dielectric layer 20 can in some embodiments be provided only in the region of the measurement points 26A and conductive paths 26B, i.e. in the region of the conductive layer 26 so as to cover and insulate the conductive layer 26. In Figure 1, a cross sectional view of a measurement point 26A of the conductive layer 26 is shown. As shown in Figure 1, the dielectric layer 20 at least partially encompasses the subjacent conductive layer 26, i.e. the measurement point 26A. The dielectric layer is also stretchable.

A recess 28 is provided in the electrode patch 10 in the area of the measurement point 26A. The recess 28 extends through the entire thickness of both the adhesive layer 12 and the dielectric layer 20 and thus exposes the subjacent measurement point 26A. The recess 28 is aligned with the measurement point 26A. Further, the recess 28 and the measurement point 26A can be concentric as shown in Figure 1. Both the recess 28 and the measurement point 26A can have circular, square, rectangular or any other suitable shape. The recess 28 and the measurement point 26A can have the same or a different shape.

The combination of layers and components in the region of the measurement point 26A forms one electrode of the electrode patch 10. Of course, the electrode patch can comprise multiple of such electrodes, e.g. 2 to 100 electrodes.

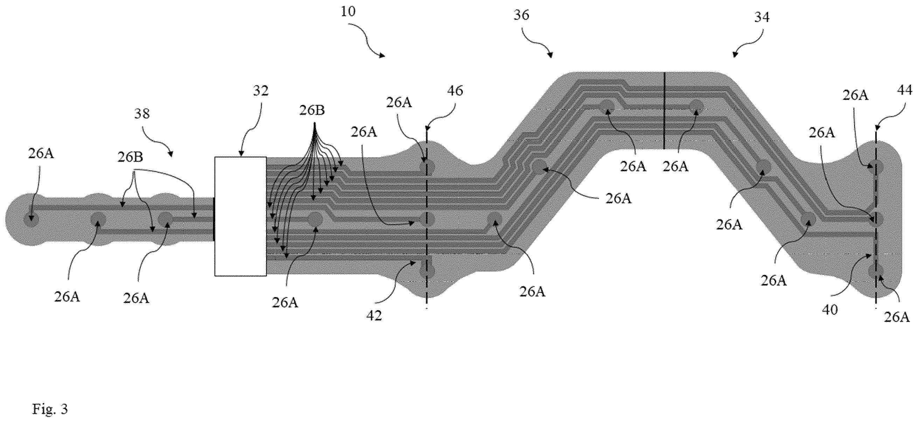

The recess 28 can receive or, as in Figure 1, is provided with an adhesive and conductive substance 30, e.g. with hydrogel 30. Alternatively or additionally, the recess 28 can be provided with one or more cover layers such as AgCl. Alternatively, in a case in which biocompatible conductive substances for the measurement point, the recess does not need to be provided with any additional substance. The substance 30 is configured to conductively connect the corresponding measurement point 26A of the conductive layer 26 to the skin of the subject surface 16 when the electrode patch 10 is applied to the subject. As the dielectric layer 20 insulates all other portions of the conductive layer 26, apart from the portion/s in the area of the recess 28, conductive connections between the subject surface 16 and the conductive layer 26 can only be established via the substance 30, i.e. via the recesses 28. The signals received by the measurement point 26A are further transmitted to a conductive path 26B conductively connected with the corresponding measurement point 26A (see Figure 3).

In the present embodiment, the conductive layer 26 is stretchable and is formed by a silver-carbon paste comprising 70wt% silver paste. This preferred composition ensures an optimal transmission of the obtained signals even when the electrode patch 10 is stretched during use.

Further, in the embodiment shown in Figure 1, the description layer 22 is provided adjacent to the conductive layer 26 in a direction oriented away from the subject surface 16 when the electrode patch 10 is applied to the subject, i.e. used in the predetermined manner. The description layer 22 can visualize the corresponding measurement point 26A. Alternatively or in addition, the description layer 22 can visualize at least one instruction and/or at least one reference point for supporting the predetermined positioning of the electrode patch 10 on the subject. It will be understood that the description layer 22 does not have to be a continuous layer, but can be present e.g. only in predetermined positions of the patch. The description layer 22 can be stretchable.

The carrier layer 24 is disposed adjacent to the description layer 22 in a direction oriented away from the subject surface 16 when the electrode patch 10 is applied to the subject. In the present example, the carrier layer 24 is a TPU layer. It provides sufficient stability and elasticity, while providing sufficient comfortability for the subject applied with the electrode patch 10, even during long-term use.

Figure 2 shows a cross sectional view of the same embodiment of the electrode patch shown in Figure 1 in the region of a conductive path 26B. It is apparent from Figure 2 that no recess is provided in the regions of conductive paths. Thus, the conductive paths of the electrode patch 10 are insulated towards the outside patch surfaces and are each only conductively connected to a corresponding measurement point and a connector 32 (see Figure 3). With other words, each conductive path 26B is only indirectly conductively connected to the subject via a measurement point 26A and substance 30.

All of the layers of the electrode patch 10 shown in Figures 1 and 2 are radiolucent, i.e. are substantially or fully transparent to electromagnetic radiation employed in common (medical) imaging procedures, such as X-ray or MRI, in a manner and degree similar to human soft tissue, e.g. muscle tissue. Thus, these layers do not interferingly emerge in X-ray or MRI pictures so that the electrode patch according to the invention can be worn by a patient during X-ray and/or MRI treatment without negatively affecting the treatment.

At least one and preferably all of the adhesive layer 12, the dielectric layer 20, the description layer 22, the carrier layer 24 and the substance 30 have greater radiolucency than the conductive layer 26. However, in the present embodiment, even the conductive layer 26 is radiolucent, i.e. does not darken an image of a common hospital X-ray (RTG) more than 50% of image intensity, preferably not more than 40%, preferably not more than 30%.

As different layer combinations are present in different regions of the electrode patch, the radiolucency can vary in different regions of the electrode patch, depending on the number, thickness and type of layers present in the particular region.

Further, in the shown embodiment, all layers except of the description layer 22 and the conductive layer 26 are optically transparent, i.e. the human eye is able to see through these layers and to see structures, items, etc. underneath the electrode patch.

An overall shape of the electrode patch according to an exemplary embodiment of the present invention is shown in Figure 3. The shape is not limited to a particular internal structure or layer combination of the electrode patch. Figure 3 shows an example of the electrode patch 10 comprising a conductive layer 26 having 16 measurement points 26A, wherein each measurement point 26A is conductively connected to the connector 32 via a corresponding conductive path 26B.

The connector 32 is adhesively attached to the carrier layer 24 in the present case. The connector 32 is not only conductively connected to the conductive paths 26B, but is also conductively connectable to a device (not shown) so that signals can be transferred from the conductive paths 26B to the device via the connector 26A. Alternatively, the device and the connector can together form an integrated component. In the present example, the connector 32 is polymer based and is radiolucent and optically transparent.

The shown electrode patch 10 comprises a first portion 34 which is designed to extend to the right side of the subject's sternal midline in an attached state of the electrode patch 10. Further, the electrode patch 10 comprises a second portion 36 which is designed to extend to the left side of the subject's sternal midline in an attached state of the electrode patch 10, and a third portion 38 which is designed to being disposed posteriorly in an attached state of the electrode patch 10.

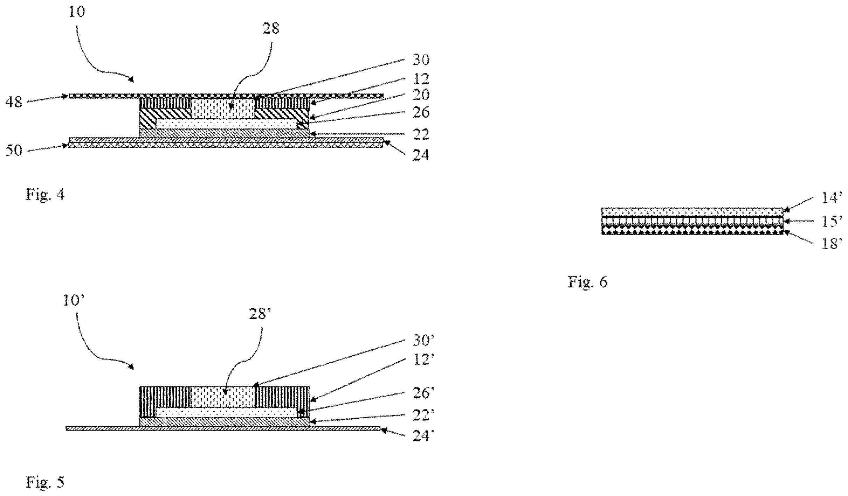

This electrode design is particularly advantageous regarding a quick and correct positioning. For applying the electrode patch 10, the user, e.g. a practitioner, can successively attach initially the first portion 34, subsequently the second portion 36 and afterwards a third portion 38 of the electrode patch 10. Thus, the shown electrode patch 10 can be placed fully and correctly in under 20 seconds (with right sided leads and posterior leads). Each of the above steps of attaching the first, second and third portions of the electrode patch 10 can include removing a corresponding first, second and third portion of an outer cover layer (see Figure 4) of the electrode patch 10, which outer cover layer can be a backing foil protecting the adhesive layer 12.

The electrodes embedded in the third portion 38 of the electrode patch 10 are able to record posterior leads, which results in higher accuracy and an increase in the number of leads to visualize ST segment changes and to diagnose a heart attack in the posterior wall or occluded right sided arteries that currently are difficult to diagnose.

It will be understood that for patients with dextrocardia the afore-described configuration can be mirrored. Also, the shape and outline of the electrode patch can be adapted specifically for males and females as electrodes for females must go beneath the breast in contrast to electrodes for males.

As can be seen in the design of the electrode patch 10 shown in Figure 3, all angles and radii of the outline of the electrode patch 10 are designed sufficiently obtuse and large. This further improves the wearing comfort for the subject the patch is applied to.

Each of the first portion 34 and the second portion 36 of the electrode patch comprises three aligned electrodes 40, 42 that are aligned along a straight line (shown by the dashed lines 44, 46). These straight lines are substantially parallel to the sternal midline of a patient in a state in which the electrode patch 10 is attached to the patient in a predetermined manner. These aligned electrodes 40, 42 are provided to obtain the limb leads during ECG. Thus, the aligned electrodes are spaced approx. 5 cm to 25 cm, preferably 10 cm to 20 cm, more preferably 12.5 cm to 17.5 cm, still more preferably about 15 cm, from a line where the first portion 34 and the second portion 36 of the electrode patch 10 are connected.

Figure 4 shows the first embodiment of the electrode patch 10 described above referring to Figures 1 and 2 during a manufacturing process. The electrode patch 10 is shown in the region of a measurement point 26A. As most of the layers shown in Figure 4 correspond to the layers shown in Figure 1, it is primarily referred to the differences between Figure 4 and Figure 1.

In addition to the layers seen in Figure 1, there is further provided an outer cover layer 48. The outer cover layer 48 is applied onto the adhesive contact surface 14 of the adhesive layer 12 so as to protect the adhesive contact surface 14, e.g. during transportation and storage. The outer cover layer 48 can be a PET liner, a backing foil, etc. The outer cover layer 48 has to be removed from the electrode patch 10 before the patch is applied to the subject. In an embodiment, the outer cover layer 48 can be separated into three outer cover layer portions (not shown) for covering each of the three electrode patch portions described above.

Further, Figure 4 shows a support layer 50 that is arranged on the electrode patch adjacent to the carrier layer 24 on a surface of the carrier layer 24 opposing the surface that faces the conductive 26 and adhesive layer 12. The support layer 50 provides support and stability to the electrode patch 10 during manufacturing is removed after manufacturing. Thus, the support layer 50 is only an auxiliary layer supporting the manufacturing of the electrode patch. The support layer 50 can be a siliconized PET layer and can be stretchable, which improves the handling during manufacturing.

Figure 5 shows a second embodiment of the electrode patch in the region of a measurement point. In contrast to the first embodiment of Figures 1 and 2, the electrode patch 10' of Figure 5 comprises an adhesive layer 12' that is dielectric. Thus, a separate dielectric layer can be omitted in this embodiment. Consequently, the electrode patch 10' has a thinner configuration than the electrode patch 10 described above. Besides this difference, the electrode patch 10' according to the second embodiment and the electrode patch 10 according to the first embodiment can be alike in its structure, configuration and function. Exemplarily, the adhesive layer 12' being dielectric can be a double sided adhesive tape.

The structure of such a double sided adhesive tape is exemplarily shown in Figure 6. As can be seen, the double sided adhesive tape comprises an adhesive contact surface 14', a structure layer 15' and an adhesive patch surface 18'. The structure layer 15' is arranged between the adhesive contact surface 14' and the adhesive patch surface 18'. The structure layer 15' can for example be a non-woven layer.

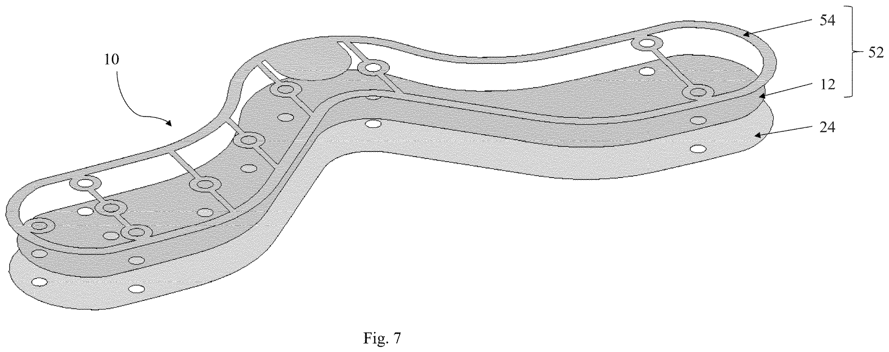

Figure 7 shows a further embodiment of the electrode patch 10, in which the electrode patch 10 comprises a hybrid adhesive layer 52 comprising the adhesive layer 12 and a reinforcing adhesive layer 54. Apart from the adhesive layers, Figure 7 only shows a simplified representation of the electrode patch 10, more precisely only shows carrier layer 24. However, the electrode patch 10 shown in Figure 7 can comprise further features discussed in the context of the other embodiments (conductive layer, measurement points, etc.).

As can be seen in Figure 7, the reinforcing adhesive layer 54 is only arranged in predetermined positions and areas of the patch 10 so as to locally reinforce the adhesiveness of the electrode patch 10 in the area of the patch edges, in the form of rings in the area of the measurement points and in the area in which a connector or device is attachable to the patch 10. The adhesive layer 12 is made of a silicone adhesive, which is highly biocompatible and gentle to the skin of a subject, wherein the reinforcing adhesive layer 54 is made of an acrylic adhesive, which has a stronger adhesiveness compared to the silicone adhesive layer.

It is to be mentioned that in other embodiments of the electrode patch according to the invention other (not shown in the Figures), some of the layers shown in the embodiments can be omitted. In particular, there can be provided an electrode patch only comprising an elastic adhesive layer and an elastic conductive layer. In this case, the adhesive layer should be dielectric. The adhesive layer can be used to apply the conductive layer to the subject. Such an electrode patch is very thin and comfortable to wear for the subject, e.g. a patient.

While the invention has been described with respect to a limited number of embodiments, it will be appreciated that many variations, modifications and other applications of the invention may be made, and that various combinations and subcombinations of embodiments are also possible and encompassed within the scope of this application.

## Claims

1. Electrode patch (10, 10'), in particular for performing electrocardiography, comprising:
an elastic adhesive layer (12, 12') having an adhesive contact surface (14, 14') that is configured to adhere to the skin of a subject the electrode patch (10, 10') is applied to;
an elastic conductive layer (26, 26') including a plurality of measurement points (26A) and conductive paths (26B), the conductive paths (26B) being configured to conductively connect the plurality of measurement points (26A) to a connector (32); and
a plurality of electrodes, wherein each of the plurality of electrodes is formed at least by one of the plurality of measurement points (26A) and a corresponding recess (28) provided in the adhesive layer (12, 12'), which recess (28) extends through the entire layer thickness of the adhesive layer (12, 12') and is provided or providable with a substance (30, 30') that is adhesive and conductive, such as hydrogel, so as to conductively connect the one of the plurality of measurement points (26A) to the skin of the subject;
wherein at least 90% of the area of the electrode patch (10, 10') is radiolucent by means of radiolucency of the adhesive layer (12, 12') and the conductive layer (26, 26'), and
wherein the electrode patch (10, 10') comprises a hybrid adhesive layer (52) comprising the adhesive layer (12), which is locally adhesively reinforced by a reinforcing adhesive layer (54).

2. Electrode patch (10, 10') according to claim 1, wherein the adhesive layer (12, 12') has an elastic modulus from 1.0 to 600.0 N/mm², preferably from 2.0 to 500.0 N/mm², more preferably from 3.0 to 250.0 N/mm², still more preferably from 3.0 to 20.0 N/mm².

3. Electrode patch (10, 10') according to claim 1 or 2, wherein the adhesive layer (12, 12') is at least partially optically transparent.

4. Electrode patch (10, 10') according to one of the preceding claims, wherein the conductive layer (26, 26') comprises a conductive particle composition, in particular a silver-carbon paste, the silver-carbon paste preferably having 50 to 90 wt% silver paste with 30 to 80% solid constituents.

5. Electrode patch (10, 10') according to one of the preceding claims, further comprising an elastic carrier layer (24, 24'), wherein the conductive layer (26, 26') is arranged between the carrier layer (24, 24') and the adhesive layer (12, 12'), and wherein the carrier layer (24, 24') is radiolucent.

6. Electrode patch (10, 10') according to one of the preceding claims, further comprising a description layer (22, 22') visualizing at least one of the plurality of measurement points (26A) and/or at least one instruction and/or at least one reference point for supporting the predetermined positioning of the electrode patch (10, 10') on the subject.

7. Electrode patch (10, 10') according to one of the preceding claims, further comprising a dielectric layer (20, 20') being arranged between the adhesive layer (12, 12') and the conductive layer (26, 26'), wherein the recess (28) also extends through the entire layer thickness of the dielectric layer (20, 20').

8. Electrode patch (10, 10') according to one of the preceding claims, wherein the adhesive layer (12, 12') is dielectric.

9. Electrode patch (10, 10') according to one of the preceding claims, wherein the electrode patch (10, 10') is partly perforated.

10. Electrode patch (10, 10') according to one of the preceding claims, wherein the electrode patch (10, 10') has a thickness between 10 and 1000 µm in the area of the at least one measurement point (26A) and a thickness between 10 and 1000 µm in the other areas, wherein preferably the thickness in the area of the at least one measurement point (26A) is larger than the thickness in the other areas.

11. Electrode patch (10, 10') according to one of the preceding claims,
wherein the electrode patch (10, 10') has a single-piece form; and/or
wherein the electrode patch (10, 10') comprises:
a first portion (34) which is designed to extend to the right side of the subject's sternal midline in an attached state of the electrode patch (10, 10'),
a second portion (36) which is designed to extend to the left side of the subject's sternal midline in an attached state of the electrode patch (10, 10'), and
a third portion (38) which is designed to being disposed posteriorly in an attached state of the electrode patch (10, 10').

12. Electrode patch (10, 10') according to one of the preceding claims,
wherein the electrode patch (10, 10') comprises the connector (32), which is conductively connected to the conductive paths (26B) and is conductively connected or connectable to an internal or external device so that signals can be transferred from the conductive paths (26B) to the device via the connector (32); and
wherein preferably the device is magnetically and/or mechanically, removably connectable to the connector (32).

13. System, in particular for performing electrocardiography, comprising:
an electrode patch (10, 10') according to one of claims 1 to 11;
a connector (32) which is conductively connected to the conductive paths (26B); and
an internal or external device which is conductively connected or connectable to the connector (32) so that signals can be transferred from the conductive paths (26B) to an internal or external device via the connector (32).

14. Method of manufacturing an electrode patch (10, 10') according to one of claims 1 to 12, the method comprising the steps:
providing an elastic adhesive layer (12, 12') having an adhesive contact surface (14, 14') that is configured to adhere to the skin of a subject the electrode patch (10, 10') is applied to;
providing a flexible conductive layer (26, 26') including a plurality of measurement points (26A) and conductive paths (26B), the conductive paths (26B) being configured to conductively connect the plurality of measurement points (26A) to a connector (32); and
embedding a plurality of electrodes in the electrode patch (10, 10'), wherein each of the plurality of electrodes is formed at least by one of the plurality of measurement points (26A) and a corresponding recess (28) provided in the adhesive layer (12, 12'), which recess (28) extends through the entire layer thickness of the adhesive layer (12, 12') and is provided or providable with a substance that is adhesive and conductive (30, 30'), such as hydrogel, so as to conductively connect the one of the plurality of measurement points (26A) to the skin of the subject;
wherein at least 90% of the area of the electrode patch (10, 10') is radiolucent by means of radiolucency of the adhesive layer (12, 12') and the conductive layer (26, 26'), and
wherein the electrode patch (10, 10') comprises a hybrid adhesive layer (52) comprising the adhesive layer (12), which is locally adhesively reinforced by a reinforcing adhesive layer (54).

## Patentansprüche

1. Elektrodenpflaster (10, 10'), insbesondere zum Durchführen von Elektrokardiographie, umfassend:
eine elastische haftende Schicht (12, 12') mit einer haftenden Kontaktfläche (14, 14'), die dazu ausgebildet ist, an der Haut eines Subjekts zu haften, an dem das Elektrodenpflaster (10, 10') angebracht ist;
eine elastische leitfähige Schicht (26, 26'), die eine Vielzahl von Messpunkten (26A) und Leiterbahnen (26B) umfasst, wobei die Leiterbahnen (26B) dazu ausgebildet sind, die Vielzahl von Messpunkten (26A) leitend mit einem Verbinder (32) zu verbinden; und
eine Mehrzahl von Elektroden, wobei jede der Mehrzahl von Elektroden wenigstens durch einen der Mehrzahl von Messpunkten (26A) und eine entsprechende, in der haftenden Schicht (12, 12') bereitgestellte Vertiefung (28) gebildet ist, wobei sich die Vertiefung (28) durch die gesamte Schichtdicke der haftenden Schicht (12, 12') erstreckt und mit einer Substanz (30, 30'), die haftend und leitfähig ist, wie beispielsweise Hydrogel, versehen oder versehbar ist, um den einen der Mehrzahl von Messpunkten (26A) mit der Haut des Subjekts leitend zu verbinden,
wobei wenigstens 90% der Fläche des Elektrodenpflasters (10, 10') mittels Strahlungsdurchlässigkeit der haftenden Schicht (12, 12') und der leitfähigen Schicht (26, 26') strahlungsdurchlässig ist, und
wobei das Elektrodenpflaster (10, 10') eine hybride haftende Schicht (52) umfasst, die die haftende Schicht (12) umfasst, die lokal durch eine verstärkende haftende Schicht (54) haftend verstärkt ist.

2. Elektrodenpflaster (10, 10') nach Anspruch 1, wobei die haftende Schicht (12, 12') einen Elastizitätsmodul von 1,0 bis 600,0 N/mm², vorzugsweise von 2,0 bis 500,0 N/mm², weiter bevorzugt von 3,0 bis 250,0 N/mm², noch weiter bevorzugt von 3,0 bis 20,0 N/mm² aufweist.

3. Elektrodenpflaster (10, 10') nach Anspruch 1 oder 2, wobei die haftende Schicht (12, 12') wenigstens teilweise optisch transparent ist.

4. Elektrodenpflaster (10, 10') nach einem der vorhergehenden Ansprüche, wobei die leitfähige Schicht (26, 26') eine leitfähige Partikelzusammensetzung, insbesondere eine Silber-Kohlenstoff-Paste, umfasst, wobei die Silber-Kohlenstoff-Paste vorzugsweise 50 bis 90 Gew.-% Silberpaste mit 30 bis 80% festen Bestandteilen aufweist.

5. Elektrodenpflaster (10, 10') nach einem der vorhergehenden Ansprüche, ferner umfassend eine elastische Trägerschicht (24, 24'), wobei die leitfähige Schicht (26, 26') zwischen der Trägerschicht (24, 24') und der haftenden Schicht (12, 12') angeordnet ist, und wobei die Trägerschicht (24, 24') strahlungsdurchlässig ist.

6. Elektrodenpflaster (10, 10') nach einem der vorhergehenden Ansprüche, ferner umfassend eine Beschreibungsschicht (22, 22'), die wenigstens einen der Mehrzahl von Messpunkten (26A) und/oder wenigstens eine Anweisung und/oder wenigstens einen Referenzpunkt zum Unterstützen der vorbestimmten Positionierung des Elektrodenpflasters (10, 10') auf dem Subjekt visualisiert.

7. Elektrodenpflaster (10, 10') nach einem der vorhergehenden Ansprüche, ferner umfassend eine zwischen der haftenden Schicht (12, 12') und der leitfähigen Schicht (26, 26') angeordnete dielektrische Schicht (20, 20'), wobei sich die Vertiefung (28) auch durch die gesamte Schichtdicke der dielektrischen Schicht (20, 20') erstreckt.

8. Elektrodenpflaster (10, 10') nach einem der vorhergehenden Ansprüche, wobei die haftende Schicht (12, 12') dielektrisch ist.

9. Elektrodenpflaster (10, 10') nach einem der vorhergehenden Ansprüche, wobei das Elektrodenpflaster (10, 10') teilweise perforiert ist.

10. Elektrodenpflaster (10, 10') nach einem der vorhergehenden Ansprüche, wobei das Elektrodenpflaster (10, 10') im Bereich des wenigstens einen Messpunkts (26A) eine Dicke zwischen 10 und 1000 µm und in den anderen Bereichen eine Dicke zwischen 10 und 1000 µm aufweist, wobei vorzugsweise die Dicke im Bereich des wenigstens einen Messpunkts (26A) größer als die Dicke in den anderen Bereichen ist.

11. Elektrodenpflaster (10, 10') nach einem der vorhergehenden Ansprüche,
wobei das Elektrodenpflaster (10, 10') eine einteilige Form hat, und/oder
wobei das Elektrodenpflaster (10, 10') umfasst:
einen ersten Abschnitt (34), der dazu ausgebildet ist, sich in einem angebrachten Zustand des Elektrodenpflasters (10, 10') zur rechten Seite der Sternum-Mittellinie des Subjekts zu erstrecken;
einen zweiten Abschnitt (36), der dazu ausgebildet ist, sich in einem angebrachten Zustand des Elektrodenpflasters (10, 10') zur linken Seite der Sternum-Mittellinie des Subjekts zu erstrecken; und
einen dritten Abschnitt (38), der dazu ausgebildet ist, in einem angebrachten Zustand des Elektrodenpflasters (10, 10') posterior angeordnet zu sein.

12. Elektrodenpflaster (10, 10') nach einem der vorhergehenden Ansprüche,
wobei das Elektrodenpflaster (10, 10') den Verbinder (32), der leitend mit den Leiterbahnen (26B) verbunden ist und leitend mit einer internen oder externen Vorrichtung verbunden oder verbindbar ist, so dass Signale von den Leiterbahnen (26B) über den Verbinder (32) an die Vorrichtung übertragen werden können, umfasst, und
wobei vorzugsweise die Vorrichtung magnetisch und/oder mechanisch lösbar mit dem Verbinder (32) verbindbar ist.

13. System, insbesondere zum Durchführen von Elektrokardiographie, umfassend:
ein Elektrodenpflaster (10, 10') nach einem der Ansprüche 1 bis 11;
einen Verbinder (32), der leitend mit den Leiterbahnen (26B) verbunden ist; und
eine interne oder externe Vorrichtung, die leitend mit dem Verbinder (32) verbunden oder verbindbar ist, so dass Signale von den Leiterbahnen (26B) über den Verbinder (32) an eine interne oder externe Vorrichtung übertragen werden können.

14. Verfahren zum Herstellen eines Elektrodenpflasters (10, 10') nach einem der Ansprüche 1 bis 12, wobei das Verfahren die Schritte aufweist:
Bereitstellen einer elastischen haftenden Schicht (12, 12') mit einer haftenden Kontaktfläche (14, 14'), die dazu ausgebildet ist, an der Haut eines Subjekts zu haften, an dem das Elektrodenpflaster (10, 10') angebracht ist;
Bereitstellen einer flexiblen leitfähigen Schicht (26, 26'), die eine Mehrzahl von Messpunkten (26A) und Leiterbahnen (26B) umfasst, wobei die Leiterbahnen (26B) dazu ausgebildet sind, die Mehrzahl von Messpunkten (26A) leitend mit einem Verbinder (32) zu verbinden; und
Einbetten einer Mehrzahl von Elektroden in das Elektrodenpflaster (10, 10'), wobei jede der Mehrzahl von Elektroden wenigstens durch einen der Mehrzahl von Messpunkten (26A) und eine entsprechende, in der haftenden Schicht (12, 12') bereitgestellte Vertiefung (28) gebildet ist, wobei die Vertiefung (28) sich durch die gesamte Schichtdicke der haftenden Schicht (12, 12') erstreckt und mit einer Substanz (30, 30'), die haftend und leitfähig ist, wie beispielsweise Hydrogel, versehen oder versehbar ist, um den einen der Mehrzahl von Messpunkten (26A) mit der Haut des Subjekts leitend zu verbinden,
wobei mindestens 90% der Fläche des Elektrodenpflasters (10, 10') mittels Strahlungsdurchlässigkeit der haftenden Schicht (12, 12') und der leitfähigen Schicht (26, 26') strahlungsdurchlässig ist, und
wobei das Elektrodenpflaster (10, 10') eine hybride haftende Schicht (52) umfasst, die die haftende Schicht (12) umfasst, die lokal durch eine verstärkende haftende Schicht (54) haftend verstärkt ist.

## Revendications

1. Patch d'électrodes (10, 10'), en particulier pour la réalisation d'une électrocardiographie, comprenant :
une couche adhésive élastique (12, 12') ayant une surface de contact adhésive (14, 14') qui est configurée pour adhérer à la peau d'un sujet sur lequel le patch d'électrodes (10, 10') est appliqué ;
une couche conductrice élastique (26, 26') comprenant une pluralité de points de mesure (26A) et de chemins conducteurs (26B), les chemins conducteurs (26B) étant configurés pour relier par conduction la pluralité de points de mesure (26A) à un connecteur (32) ; et
une pluralité d'électrodes, dans laquelle chacune de la pluralité d'électrodes est formée au moins par l'un de la pluralité de points de mesure (26A) et un renfoncement correspondant (28) prévu dans la couche adhésive (12, 12'), lequel renfoncement (28) s'étend sur toute l'épaisseur de la couche adhésive (12, 12') et est pourvue ou peut être pourvue d'une substance (30, 30') adhésive et conductrice, telle qu'un hydrogel, afin de connecter de manière conductrice l'un de la pluralité de points de mesure de la pluralité (26A) à la peau du sujet ;
dans lequel au moins 90 % de la surface du patch d'électrodes (10, 10') est radiotransparente grâce à radiotransparence de la couche adhésive (12, 12') et de la couche conductrice (26, 26'), et.
dans lequel le patch d'électrode (10, 10') comprend une couche adhésive hybride (52) comprenant la couche adhésive (12), qui est localement renforcée par une couche adhésive de renfort (54).

2. Patch d'électrodes (10, 10') selon la revendication 1, dans lequel la couche adhésive (12, 12') a un module d'élasticité de 1,0 à 600,0 N/mm², de préférence de 2,0 à 500,0 N/mm², plus préférentiellement de 3,0 à 250,0 N/mm², encore plus préférentiellement de 3,0 à 20,0 N/mm².

3. Patch d'électrodes (10, 10') selon la revendication 1 ou 2, dans lequel la couche adhésive (12, 12') est au moins partiellement optiquement transparente.

4. Patch électrodes (10, 10') selon l'une des revendications précédentes, dans lequel la couche conductrice (26, 26') comprend une composition de particules conductrices, en particulier une pâte argent-carbone, la pâte argent-carbone ayant de préférence 50 à 90 % en poids de pâte d'argent avec 30 à 80 % de constituants solides.

5. Patch d'électrodes (10, 10') selon l'une des revendications précédentes, comprenant en outre une couche support élastique (24, 24'), dans laquelle la couche conductrice (26, 26') est disposée entre la couche support (24, 24') et la couche adhésive (12, 12'), et dans laquelle la couche support (24, 24) est radiotransparente.

6. Patch d'électrodes (10, 10') selon l'une des revendications précédentes, comprenant en outre une couche de description (22, 22) visualisant au moins l'un de la pluralité de points de mesure (26A) et/ou au moins une instruction et/ou au moins un point de référence pour soutenir le positionnement prédéterminé du patch à électrodes (10, 10) sur le sujet.

7. Patch d'électrodes (10, 10') selon l'une des revendications précédentes, comprenant en outre une couche diélectrique (20, 20') étant disposée entre la couche adhésive (12, 12') et la couche conductrice (26, 26'), dans lequel le renfoncement (28) s'étend également à travers toute l'épaisseur de la couche diélectrique (20, 20).

8. Patch d'électrodes (10, 10') selon l'une des revendications précédentes, dans lequel la couche adhésive (12, 12') est diélectrique.

9. Patch d'électrodes (10, 10') selon l'une des revendications précédentes, dans lequel le patch d'électrodes (10, 10) est partiellement perforé.

10. Pièce d'électrodes (10, 10') selon l'une des revendications précédentes, dans lequel le patch d'électrodes (10, 10) a une épaisseur comprise entre 10 et 1000 µm dans la zone du au moins un point de mesure (26A) et une épaisseur comprise entre 10 et 1000 µm dans les autres zones, dans lequel de préférence l'épaisseur dans la zone du au moins un point de mesure (26A) est plus grande que l'épaisseur dans les autres zones.

11. Patch d'électrode (10, 10') selon l'une des revendications précédentes,
dans lequel le patch d'électrodes (10, 10') a une forme monobloc ; et/ou
dans lequel le patch d'électrodes (10, 10') comprend :
une première partie (34) qui est conçue pour s'étendre sur le côté droit de la ligne médiane sternale du sujet dans un état de fixation du patch d'électrodes (10, 10'),
une deuxième partie (36) conçue pour s'étendre sur le côté gauche de la ligne médiane sternale du sujet dans un état de fixation du patch d'électrodes (10, 10'), et
une troisième partie (38) conçue pour être disposée postérieurement dans un état de fixation du patch d'électrodes (10, 10).

12. Patch d'électrodes (10, 10') selon l'une des revendications précédentes,
dans lequel le patch d'électrodes (10, 10') comprend le connecteur (32), qui est connecté de manière conductrice aux chemins conducteurs (26B) et connecté ou connectable de manière conductrice à un dispositif interne ou externe de manière à ce que les signaux puissent être transférés des chemins conducteurs (26B) au dispositif par l'intermédiaire du connecteur (32) ; et
dans lequel, de préférence, le dispositif est magnétiquement et/ou mécaniquement, connectable de manière amovible au connecteur (32).

13. Système, en particulier pour la réalisation d'une électrocardiographie, comprenant :
un patch d'électrodes (10, 10') selon l'une des revendications 1 à 11 ;
un connecteur (32) qui est connecté de manière conductrice aux chemins conducteurs (26B) ; et
un dispositif interne ou externe connecté ou connectable de manière conductrice au connecteur (32) de manière à ce que les signaux puissent être transférés des chemins conducteurs (26B) à un dispositif interne ou externe par l'intermédiaire du connecteur (32).

14. Procédé de fabrication d'un patch d'électrodes (10, 10') selon l'une des revendications 1 à 12, le procédé comprenant les étapes :
fournir une couche adhésive élastique (12, 12') ayant une surface de contact adhésive (14, 14') configurée pour adhérer à la peau d'un sujet sur lequel le patch d'électrode (10, 10') est appliqué ;
fournir une couche conductrice flexible (26, 26') comprenant une pluralité de points de mesure (26A) et des chemins conducteurs (26B), les chemins conducteurs (26B) étant configurés pour connecter de manière conductrice la pluralité de points de mesure (26A) à un connecteur (32) ; et
incorporer une pluralité d'électrodes dans le patch d'électrodes (10, 10'), dans lequel chacune des pluralité d'électrodes est formée au moins par l'un de la pluralité de points de mesure (26A) et par un renfoncement (28) correspondant dans la couche adhésive (12, 12'), lequel renfoncement (28) s'étend sur toute l'épaisseur de la couche adhésive (12, 12') et est pourvu ou peut être pourvu d'une substance adhésive et conductrice (30, 30'), telle qu'un hydrogel, afin de connecter de manière conductrice l'un de la pluralité de points de mesure (26A) à la peau du sujet ;.
dans lequel au moins 90 % de la surface du patch d'électrode (10, 10') est radiotransparente grâce à la radiotransparence de la couche adhésive (12, 12') et de la couche conductrice (26, 26'), et
dans lequel le patch d'électrodes (10, 10') comprend une couche adhésive hybride (52) comprenant la couche adhésive (12), qui est localement renforcée par adhésif par une couche adhésive de renforcement (54).
